(19) Europäisches Patentamt
European Patent Office
Office européen des brevets

(11) Veröffentlichungsnummer: **0 622 368 A1**

(12) # EUROPÄISCHE PATENTANMELDUNG

(21) Anmeldenummer: **94105775.4**

(22) Anmeldetag: **14.04.94**

(51) Int. Cl.5: **C07D 491/04**, A61K 31/47, C07D 401/04, C07D 401/14, C07D 417/14, //(C07D491/04, 307:00,221:00)

(30) Priorität: **27.04.93 DE 4313697**

(43) Veröffentlichungstag der Anmeldung: **02.11.94 Patentblatt 94/44**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Stoltefuss, Jürgen, Dipl.-Ing.**
**Parkstrasse 20**
**D-42781 Haan (DE)**
Erfinder: **Goldmann, Siegfried, Dr.**
**Am Osterholz 91**
**D-42327 Wuppertal (DE)**
Erfinder: **Straub, Alexander, Dr.**
**Moospfad 30**
**D-42113 Wuppertal (DE)**
Erfinder: **Bechem, Martin, Dr.**
**Hans-Böckler-Strasse 102**
**D-42111 Wuppertal (DE)**
Erfinder: **Gross, Rainer, Prof. Dr.**
**Platzhofstrasse 23**
**D-42115 Wuppertal (DE)**
Erfinder: **Hebisch, Siegbert, Dr.**
**Johann-Breuker-Platz 8**
**D-46244 Bottrop (DE)**
Erfinder: **Hütter, Joachim, Dr.**
**Teschen-Sudberger-Strasse 13**
**D-42349 Wuppertal (DE)**
Erfinder: **Rounding, Howard-Paul, Dr.**
**Pahlkestrasse 15**
**D-42115 Wuppertal (DE)**

(54) **Chinolyl-dihydropyridinester zur Behandlung von Herz-Kreislauferkrankungen.**

(57) Die Erfindung betrifft neue 4-Chinolyl-dihydropyridinester der allgemeinen Formel (I)

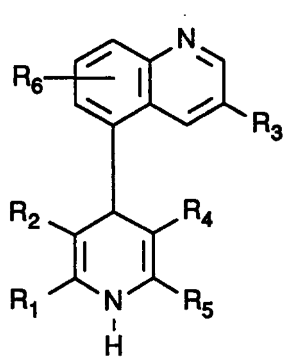

(I),

in welcher

$R_1$ bis $R_6$ die in der Beschreibung angegebene Bedeutung haben,

Verfahren zu ihrer Herstellung und ihre Verwendung in Arzneimitteln, insbesondere in Mitteln zur Behandlung von Herz-Kreislauf-Erkrankungen.

EP 0 622 368 A1

Die Erfindung betrifft neue 4-Chinolyl-dihydropyridinester, Verfahren zu ihrer Herstellung und ihre Verwendung in Arzneimitteln, insbesondere in Mitteln zur Behandlung von Herz-Kreislauferkrankungen.

Es ist bereits bekannt, daß 1,4-Dihydropyridine gefäßerweiternde Eigenschaften besitzen und als Coronarmittel und Antihypertensiva verwendet werden können. Weiterhin ist bekannt, daß 1,4-Dihydropyridine eine Hemmung der Kontraktionskraft von glatten und cardialen Muskeln bewirken und zur Behandlung von Coronar- und Gefäßerkrankungen eingesetzt werden können. Aus US 5 100 900 sind auch bereits 4-Chinolyl-dihydropyridine bekannt, die eine positiv inotrope Wirkung besitzen.

Bei Kenntnis des Standes der Technik war es nicht vorhersehbar, daß die erfindungsgemäßen Verbindungen eine kontraktionskraftverstärkende, am Herzmuskel positiv inotrope Wirkung mit weitgehend gefäßneutralem Verhalten besitzen.

Die vorliegende Erfindung betrifft neue 4-Chinolyl-dihydropyridinester der allgemeinen Formel (I)

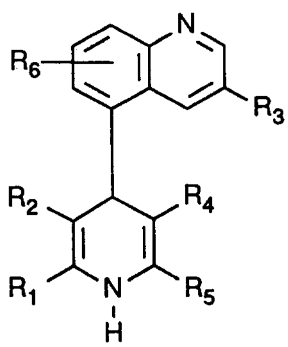

(I)

in welcher

$R^1$ und $R^5$ gleich oder verschieden sind und für geradkettiges oder verzweigtes Alkyl mit bis zu 8 Kohlenstoffatomen stehen,

$R^2$ für Nitro oder Cyano steht,

oder

$R^1$ und $R^2$ gemeinsam einen Lactonring der Formel

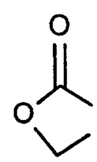

bilden,

worin

$R^6$ für Wasserstoff, Halogen oder geradkettiges oder verzweigtes Alkyl oder Alkoxy mit jeweils bis zu 8 Kohlenstoffatomen steht,

$R^3$ für Aryl mit 6 bis 10 Kohlenstoffatomen steht, das gegebenenfalls bis zu 2-fach gleich oder verschieden durch Halogen, Nitro, Cyano, Hydroxy, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder für
Thienyl oder Pyridyl steht, die gegebenenfalls durch Halogen substituiert sind,

$R^4$ für Cyano, Nitro oder Formyl steht

oder

$R^4$ und $R^5$ gemeinsam einen Lactonring der Formel

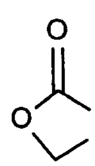

bilden,

oder

$R^4$ für eine Gruppe der Formel - CO-A-$R^8$ oder - CO-$NR^9R^{10}$ steht,

worin

A eine direkte Bindung oder ein Sauerstoffatom bedeutet,

$R^8$ Cycloalkyl mit 3 bis 8 Kohlenstoffatomen bedeutet, das gegebenenfalls durch die Gruppe - $NR^{11}$ unterbrochen ist, oder einen geradkettigen, verzweigten, cyclischen, gesättigten oder ungesättigten Kohlenwasserstoffrest mit bis zu 12 Kohlenstoffatomen bedeutet, der gegebenenfalls durch Sauerstoff,

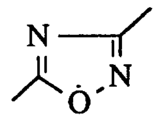

oder durch Aryliden mit 6 bis 10 Kohlenstoffatomen unterbrochen ist, wobei das Aryliden gegebenenfalls durch Halogen, Nitro, Amino, Hydroxy, Carboxy oder durch geradkettiges oder verzweigtes Alkyl, Alkoxy oder Alkoxycarbonyl mit jeweils bis zu 8 Kohlenstoffatomen substituiert ist oder der Kohlenwasserstoffrest gegebenenfalls durch Phenyl substituiert oder ersetzt ist, das seinerseits durch Fluor, Chlor, Methyl oder Methoxy substituiert sein kann,

oder der Kohlenwasserstoffrest gegebenenfalls durch eine Gruppe der Formel -$S(O)_a$ oder - $NR^{11}$ unterbrochen ist,

a eine Zahl 0, 1 oder 2 bedeutet

und

$R^{11}$ Wasserstoff oder Aryl mit 6 bis 10 Kohlenstoffatomen bedeutet, das gegebenenfalls durch Halogen, Nitro, Phenyl oder geradkettiges oder verzweigtes Alkyl oder Alkoxy mit jeweils bis zu 4 Kohlenstoffatomen substituiert ist,

oder geradkettiges oder verzweigtes Alkyl mit bis zu 6 Kohlenstoffatomen oder Cycloalkyl mit 3 bis 8 Kohlenstoffatomen bedeutet, wobei sowohl Alkyl als auch Cycloalkyl gegebenenfalls durch Aryl mit 6 bis 10 Kohlenstoffatomen substituiert sind

und wobei der Kohlenwasserstoffrest im Fall, daß A für Sauerstoff steht, immer ein-oder zweifach gleich oder verschieden substituiert ist durch Cycloalkyl mit 3 bis 8 Kohlenstoffatomen oder durch -CO-$NR^{12}R^{13}$, - $NR^{14}$-CO-$R^{15}$, - $NR^{16}$-$SO_2$-$R^{17}$, - $SO_2$-$NR^{18}R^{19}$, O-$NO_2$, - O-$(CH_2)_b$-$R^{20}$, - $S(O)_c$-$(CH_2)_d$-$R^{21}$, - $NR^{22}R^{23}$ oder -$NR^{24}COOR^{25}$,

worin

$R^{12}$, $R^{13}$, $R^{14}$, $R^{15}$, $R^{16}$, $R^{17}$, $R^{18}$, $R^{19}$, $R^{24}$ und $R^{25}$ gleich oder verschieden sind und die oben angegebene Bedeutung von $R^{11}$ haben und mit dieser gleich oder verschieden sind,

b eine Zahl 1, 2, 3, 4 oder 5 bedeutet,

d eine Zahl 0, 1, 2, 3, 4 oder 5 bedeutet,

c — die oben angegebene Bedeutung von a hat und mit dieser gleich oder verschieden ist,

$R^{20}$ und $R^{21}$ — gleich oder verschieden sind und Aryl mit 6 bis 10 Kohlenstoffatomen bedeuten, das gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert ist,

$R^{22}$ und $R^{23}$ — gleich oder verschieden sind und Wasserstoff oder einen geradkettigen, verzweigten, gesättigen, ungesättigen oder cyclischen Kohlenwasserstoffrest mit bis zu 8 Kohlenstoffatomen bedeuten, der gegebenenfalls bis zu 2-fach gleich oder verschieden durch Hydroxy, Halogen, Cycloalkyl mit 3 bis 6 Kohlenstoffatomen oder durch Aryl oder Aryloxy mit 6 bis 10 Kohlenstoffatomen substituiert ist, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können, oder

Aryl mit 6 bis 10 Kohlenstoffatomen bedeuten, das gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert ist,

oder

$R^{22}$ und $R^{23}$ — gemeinsam mit dem Stickstoffatom einen 5-bis 7-gliedrigen, gesättigen oder ungesättigten Heterocyclus mit bis zu 3 Heteroatomen aus der Reihe S, N oder O bilden, der gegebenenfalls durch geradkettiges oder verzweigtes Alkyl mit bis zu 4 Kohlenstoffatomen substituiert ist, das seinerseits durch Phenyl substituiert sein kann, oder gegebenenfalls durch Phenyl substituiert ist, das seinerseits durch Halogen substituiert sein kann,

oder der Kohlenwasserstoffrest im Fall, daß A für Sauerstoff steht, substituiert ist durch einen 3-bis 7-gliedrigen gesättigten oder ungesättigten Heterocyclus oder Heterocyclyloxyring, der bis zu 3 Heteroatome aus der Reihe S, N oder O oder Gruppen der Formel -CO oder - $SO_2$ enthalten kann,

wobei der Heterocyclus seinerseits bis zu 2-fach gleich oder verschieden durch Halogen, Hydroxy oder durch Aryl oder Arylsulfonyl mit 6 bis 10 Kohlenstoffatomen substituiert sein kann, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können,

oder der Heterocyclus gegebenenfalls bis zu 2-fach gleich oder verschieden durch geradkettiges oder verzweigtes Alkyl, Alkoxy oder Alkenyl mit jeweils bis zu 8 Kohlenstoffatomen substituiert ist, die ihrerseits bis zu 2-fach gleich oder verschieden

durch - $NR^{26}R^{27}$ substituiert sein können,
worin

$R^{26}$ und $R^{27}$ die oben angegebene Bedeutung von $R^{22}$ und $R^{23}$ haben und mit diesen gleich oder verschieden sind,
und die gesamten Alkyl-und Alkenyl-Reste gegebenenfalls ihrerseits durch Phenyl oder Phenoxy substituiert sind, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 4 Kohlenstoffatomen substituiert sein können,

$R^9$ und $R^{10}$ gleich oder verschieden sind und Wasserstoff, einen geradkettigen, verzweigten, cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5-bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,
oder

$R^9$ und $R^{10}$ gemeinsam unter Einbezug des Stickstoffatoms einen 3-bis 8-gliedrigen, gesättigen oder ungesättigten Heterocyclus bilden, der gegebenenfalls durch Sauerstoff oder durch einen Rest der Formel $S(O)_e$, - CO-oder - $NR^{28}$ unterbrochen sein kann,
worin

e eine Zahl 0, 1 oder 2 bedeutet,

$R^{28}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist, oder
einen cyclischen, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5-bis 7-gliedrigen, gesättigten

oder ungesättigten Heterocyclus mit 3 Heteroatomen aus der Reihe S, N oder O substituiert ist, die ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, und der gegebenenfalls durch geradkettiges oder verzweigtes Alkxoy oder Alkylthio mit jeweils bis zu 4 Kohlenstoffatomen, Halogen, Aryl mit 6 bis 10 Kohlenstoffatomen, einen 5-bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert ist,

und deren Salze.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),
in welcher

$R^1$ und $R^5$ gleich oder verschieden sind und für geradkettiges oder verzweigtes Alkyl mit bis zu 4 Kohlenstoffatomen stehen,

$R^2$ für Nitro oder Cyano steht,

oder

$R^1$ und $R^2$ gemeinsam einen Lactonring der Formel

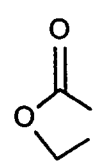

bilden,

$R^6$ für Wasserstoff, Fluor oder Chlor steht,

$R^3$ für Phenyl steht, das gegebenenfalls bis zu zweifach durch Fluor, Chlor, Brom, Nitro, Cyano, Hydroxy, Trifluormethyl oder durch geradkettiges oder verzweigtes Alkyl, Alkoxy oder Alkoxycarbonyl mit jeweils bis zu 4 Kohlenstoffatomen substituiert ist, oder für Thienyl oder Pyridyl steht, die gegebenenfalls durch Fluor, Chlor oder Brom substituiert sind,

$R^4$ für Cyano, Nitro oder Formyl steht

oder

$R^4$ und $R^5$ gemeinsam einen Lactonring der Formel

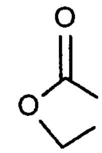

bilden,

oder

$R^4$ für eine Gruppe der Formel - CO-A-$R^8$ oder - CO-$NR^9R^{10}$ steht,

worin

A eine direkte Bindung oder ein Sauerstoffatom bedeutet,

$R^8$ Cyclopropyl, Cyclobutyl, Cyclopentyl, Cyclohexyl oder Cycloheptyl bedeutet, die gegebenenfalls durch die Gruppe - $NR^{11}$ unterbrochen sind, oder einen geradkettigen, verzweigten, cyclischen, gesättigten oder ungesättigten Kohlenwasserstoffrest mit bis zu 10 Kohlenstoffatomen bedeutet, der gegebenenfalls durch Phenyl substituiert ist, und der gegebenenfalls durch Sauerstoff, Phenyliden oder durch eine Gruppe der Formel

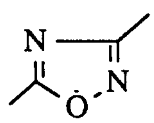 ,

$-S(O)_a$ oder - $NR^{11}$ unterbrochen ist,

worin

a eine Zahl 0,1 oder 2 bedeutet

und

$R^{11}$ Wasserstoff oder Phenyl bedeutet, das gegebenenfalls durch Halogen, Methyl, Methoxy oder Nitro substituiert ist, oder Cyclopropyl, Cyclopentyl, Cyclohexyl oder geradkettiges oder verzweigtes Alkyl mit bis zu 5 Kohlenstoffatomen bedeutet, das gegebenenfalls durch Phenyl substituiert ist,

und wobei der Kohlenwasserstoffrest im Fall, daß A für - Sauerstoff steht, immer substituiert ist durch Cyclopropyl, Cyclobutyl, Cyclopentyl, Cyclohexyl oder durch eine Gruppe - CO-$NR^{12}R^{13}$, -$NR^{14}$-CO-$R^{15}$, - $NR^{16}$-$SO_2$-$R^{17}$, - $SO_2$-$NR^{18}R^{19}$, O-$NO_2$, -O-$(CH_2)_b$-$R^{20}$, - $S(O)_c$-$(CH_2)_d$-$R^{21}$, - $NR^{22}R^{23}$ oder - $NR^{24}$-$COOR^{25}$,

worin

$R^{12}$, $R^{13}$, $R^{14}$, $R^{15}$, $R^{16}$, $R^{17}$, $R^{18}$, $R^{19}$, $R^{24}$ und $R^{25}$ gleich oder verschieden sind und die oben angegebene Bedeutung von $R^{11}$ haben und mit dieser gleich oder verschieden sind,

b eine Zahl 1, 2, 3 oder 4 bedeutet,

d eine Zahl 0, 1, 2, 3 oder 4 bedeutet,

c die oben angegebene Bedeutung von a hat und mit dieser gleich oder verschieden ist,

$R^{20}$ und $R^{21}$ gleich oder verschieden sind und Phenyl bedeuten, das gegebenenfalls bis zu 2-fach gleich oder verschieden durch Fluor, Chlor, Brom, Nitro, Hydroxy, Carboxy oder durch geradkettiges oder verzweigtes

Alkyl, Alkoxy oder Alkoxycarbonyl mit jeweils bis zu 4 Kohlenstoffatomen substituiert ist,

$R^{22}$ und $R^{23}$ gleich oder verschieden sind und Wasserstoff oder einen geradkettigen, verzweigten, gesättigen, ungesättigen oder cyclischen Kohlenwasserstoffrest mit bis zu 6 Kohlenstoffatomen bedeuten, der gegebenenfalls bis zu 2-fach gleich oder verschieden durch Fluor, Chlor, Cyclopropyl, Cyclopentyl, Cyclohexyl, Phenyl oder Phenoxy substituiert ist, die ihrerseits durch Fluor, Chlor, Brom, Nitro, Amino, Hydroxy, Carboxy oder durch geradkettiges oder verzweigtes Alkyl, Alkoxy oder Alkoxycarbonyl mit jeweils bis zu 4 Kohlenstoffatomen substituiert sein können, oder

Phenyl bedeuten, das gegebenenfalls durch Fluor, Chlor, Hydroxy, Carboxy oder durch geradkettiges oder verzweigtes Alkyl, Alkoxy oder Alkoxycarbonyl mit jeweils bis zu 3 Kohlenstoffatomen substituiert ist,

oder

$R^{22}$ und $R^{23}$ gemeinsam mit dem Stickstoffatom einen Morpholin-, Piperidin-oder Piperazinring bilden, die gegebenenfalls durch geradkettiges oder verzweigtes Alkyl mit bis zu 4 Kohlenstoffatomen, Phenyl oder Benzyl substituiert sind,

oder der Kohlenwasserstoffrest im Falle, daß A für Sauerstoff steht, substituiert ist durch Pyridyl, Tetrahydropyranyl, Pyrazolyl, Furyl, Chromanyl, Piperazinyl, Piperidinyl, Tetrahydroisochinolidinyl oder durch einen Rest der Formel

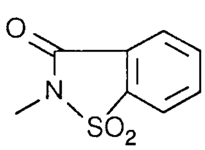 , 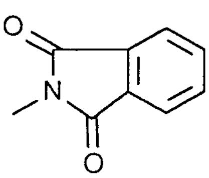

oder

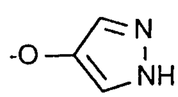

wobei die Heterocyclen ihrerseits durch Fluor, Chlor, geradkettiges oder verzweigtes Alkyl oder Alkoxy mit jeweils bis zu 3 Kohlenstoffatomen oder durch Phenyl, Benzyl oder Phenylsulfonyl substituiert sein können, die ihrerseits durch Fluor, Chlor, Brom, Nitro, Amino, Hydroxy, Carboxy oder durch geradkettiges oder verzweigtes Alkyl, Alkoxy oder Alkoxycarbonyl mit jeweils bis zu 3 Kohlenstoffatomen substituiert sein können,

$R^9$ und $R^{10}$ gleich oder verschieden sind und Wasserstoff oder einen geradkettigen, verzweigten, cyclischen, gesättigten oder ungesättigten Kohlenwasserstoffrest mit bis zu 6 Kohlenstoffatomen bedeuten, der gegebenenfalls durch Fluor, Chlor oder Phenyl substituiert ist, oder

Phenyl bedeuten, das gegebenenfalls durch Fluor, Chlor, geradkettiges oder verzweigtes Alkyl, Alkoxy, Alkylthio, Alkoxycarbonyl mit jeweils bis zu 6 Kohlenstoffatomen, Trifluormethyl oder Trifluormethoxy substituiert ist,

oder

$R^9$ und $R^{10}$ gemeinsam unter Einbezug des Stickstoffatoms einen 5-bis 6-gliedrigen, gesättigen oder ungesättigten Heterocyclus bilden, der gegebenenfalls durch ein Sauerstoffatom oder durch einen Rest der Formel - $NR^{28}$ unterbrochen sein kann,

worin

$R^{28}$ Wasserstoff, Phenyl oder einen cyclischen, geradkettigen oder verzweigten Kohlenwasserstoffrest mit bis zu 6 Kohlenstoffatomen bedeutet,

und deren Salze.

Besonders bevorzugt sind Verbindungen der allgemeinen Formel (I),

in welcher

$R^1$ und $R^5$ gleich oder verschieden sind und für Methyl oder Ethyl stehen,

$R^2$ für Nitro oder Cyano steht,

oder

$R^1$ und $R^2$ gemeinsam einen Lactonring der Formel

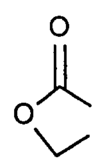

bilden,

$R^6$ für Wasserstoff steht,

$R^3$ für Phenyl steht, das gegebenenfalls durch Fluor, Chlor, Nitro, Cyano, Hydroxy, Trifluormethyl, Methyl, Ethyl, Methoxy oder Ethoxy substituiert ist,

$R^4$ für Cyano, Nitro oder Formyl steht

oder

$R^4$ und $R^5$ gemeinsam einen Lactonring der Formel

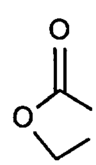

bilden,

oder

$R^4$ für eine Gruppe der Formel - CO-A-$R^8$ oder - CO-$NR^9R^{10}$ steht,

worin

A eine direkte Bindung oder ein Sauerstoffatom bedeutet,

$R^8$ Cyclopropyl, Cyclobutyl, Cyclopentyl oder Cyclohexyl bedeutet, die gegebenenfalls durch die Gruppe - $NR^{11}$ unterbrochen sind, oder

einen geradkettigen, verzweigten, cyclischen, gesättigten oder ungesättigten substituierten Kohlenwasserstoffrest mit bis zu 8 Kohlenstoffatomen bedeutet, der gegebenenfalls durch ein Sauerstoffatom, Phenyliden oder durch eine Gruppe der Formel $-S(O)_a$ oder - $NR^{11}$ unterbrochen ist und der gegebenenfalls durch Phenyl substituiert ist,

worin

a — eine Zahl 0 oder 2 bedeutet

und

$R^{11}$ — Wasserstoff oder Phenyl bedeutet, das gegebenenfalls durch Fluor, Chlor, Methyl oder Methoxy substituiert ist, oder

Cyclopropyl, Cyclopentyl, Cyclohexyl, oder geradkettiges oder verzweigtes Alkyl mit bis zu 4 Kohlenstoffatomen bedeutet, das gegebenenfalls durch Phenyl substituiert ist

und wobei der Kohlenwasserstoffrest im Falle, daß A für Sauerstoff steht, immer substituiert ist durch Cyclopropyl, Cyclobutyl, Cyclopentyl, Cyclohexyl oder durch eine Gruppe - $CO-NR^{12}R^{13}$, $-NR^{14}-CO-R^{15}$, - $NR^{16}-SO_2-R^{17}$, - $SO_2-NR^{18}R^{19}$, $O-NO_2$, $-O-(CH_2)_b-R^{20}$, - $S(O)_c-(CH_2)_d-R^{21}$, - $NR^{22}R^{23}$ oder - $NR^{24}-COOR^{25}$,

worin

$R^{12}$, $R^{13}$, $R^{14}$, $R^{15}$, $R^{16}$, $R^{17}$, $R^{18}$, $R^{19}$, $R^{24}$ und $R^{25}$ — gleich oder verschieden sind und die oben angegebene Bedeutung von $R^{11}$ haben und mit dieser gleich oder verschieden sind,

b — eine Zahl 1, 2, 3 oder 4 bedeutet,

d — eine Zahl 0, 1, 2, 3 oder 4 bedeutet,

c — die oben angegebene Bedeutung von a hat und mit dieser gleich oder verschieden ist,

$R^{20}$ und $R^{21}$ — gleich oder verschieden sind und Phenyl bedeuten, das gegebenenfalls durch Fluor, Chlor, Methyl oder Methoxy substituiert ist,

$R^{22}$ und $R^{23}$ — gleich oder verschieden sind und Wasserstoff oder einen geradkettigen, verzweigten, gesättigen, ungesättigen oder cyclischen Kohlenwasserstoffrest mit bis zu 4 Kohlenstoffatomen bedeuten, der gegebenenfalls durch Fluor, Chlor, Cyclopropyl, Cyclopentyl, Cyclohexyl, Phenyl oder Phenoxy substituiert ist, die ihrerseits durch Fluor, Chlor, Hydroxy oder durch Methyl oder Methoxy substituiert sein können, oder

Phenyl bedeuten, das gegebenenfalls durch Fluor, Chlor, Methyl oder Methoxy substituiert ist,

oder

$R^{22}$ und $R^{23}$ — gemeinsam mit dem Stickstoffatom einen Piperidin- oder Piperazinring bilden, der gegebenenfalls durch Benzyl substituiert ist,

oder der Kohlenwasserstoffrest im Falle, daß A für Sauerstoff steht, substituiert ist durch Pyridyl, Tetrahydropyranyl, Pyrazolyl, Furyl, Chromanyl, Piperazinyl, Piperidinyl, Isochinolidinyl oder durch einen Rest der Formel

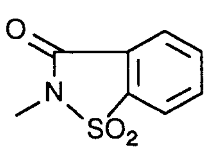 oder 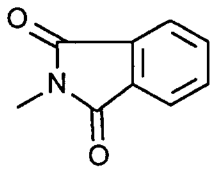

$R^9$ und $R^{10}$ gleich oder verschieden sind und Wasserstoff, einen geradkettigen, verzweigten, cyclischen, gesättigten oder ungesättigten Kohlenwasserstoffrest mit bis zu 6 Kohlenstoffatomen bedeuten, der gegebenenfalls durch Phenyl substituiert sein kann, oder Phenyl oder Pyridyl bedeuten,

und deren Salze.

Ganz besonders bevorzugt sind Verbindungen der allgemeinen Formel (I),
in welcher

$R^1$ und $R^5$ für Methyl stehen,
$R^2$ für Cyano oder Nitro steht
oder
$R^1$ und $R^2$ gemeinsam einen Lactonring der Formel

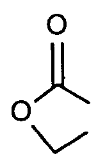

bilden,
$R^6$ für Wasserstoff steht,
und
$R^3$ für Phenyl steht, das gegebenenfalls durch Fluor, Chlor, Hydroxy, Methyl oder Methoxy substituiert ist,
$R^4$ für einen Rest der Formel - CO-A-$R^8$ oder -CO-$NR^9R^{10}$ steht,
worin
A eine direkte Bindung oder ein Sauerstoffatom bedeutet,
$R^8$ Cyclopropyl, Cyclobutyl, Cyclopentyl oder Cyclohexyl bedeutet, die gegebenenfalls durch die Gruppe - $NR^{11}$ unterbrochen sind, oder einen geradkettigen, verzweigten, cyclischen, gesättigten oder ungesättigten substituierten Kohlenwasserstoffrest mit bis zu 6 Kohlenstoffatomen bedeutet, der gegebenenfalls durch ein Sauerstoff-oder Schwefelatom oder die Gruppe - $NR^{11}$ unterbrochen ist,
worin
$R^{11}$ Methyl, Phenyl oder Benzyl bedeutet,
und wobei der Kohlenwasserstoffrest im Falle, daß A für Sauerstoff steht, immer substituiert ist durch Pyridyl, Cyclopropyl, Cyclobutyl, Cyclopentyl, Cyclohexyl oder durch eine Gruppe - $NR^{14}$-CO-$R^{15}$, - $NR^{16}$-$SO_2$-$R^{17}$, - O-$(CH_2)_b$-$R^{20}$, -$NR^{22}R^{23}$, - $NR^{24}$-$COOR^{25}$

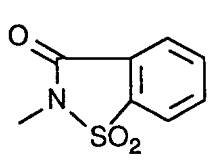 oder 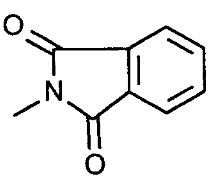

worin

| | |
|---|---|
| $R^{14}$, $R^{15}$, $R^{16}$, $R^{17}$, $R^{24}$ und $R^{25}$ | gleich oder verschieden sind und Wasserstoff oder Phenyl bedeuten, das gegebenenfalls durch Fluor, Chlor, Methyl oder Methoxy substituiert ist, oder geradkettiges oder verzweigtes Alkyl mit bis zu 4 Kohlenstoffatomen bedeuten, das gegebenenfalls durch Phenyl substituiert ist, |
| b | eine Zahl 1 oder 2 bedeutet, |
| $R^{20}$ | Phenyl bedeutet, das gegebenenfalls durch Methyl oder Methoxy substituiert ist, |
| $R^{22}$ und $R^{23}$ | gleich oder verschieden sind und Wasserstoff, Phenyl oder einen geradkettigen oder verzweigten Kohlenwasserstoffrest mit bis zu 4 Kohlenstoffatomen bedeuten, der gegebenenfalls durch Cyclopropyl, Cyclopentyl Cyclohexyl, Phenyl oder Phenoxy substituiert ist, die ihrerseits durch Hydroxy, Methyl oder Methoxy substituiert sein können, oder |
| $R^{22}$ und $R^{23}$ | gemeinsam mit dem Stickstoffatom einen Piperidinring bilden, der gegebenenfalls durch Benzyl substituiert ist, |
| $R^9$ und $R^{10}$ | gleich oder verschieden sind und Wasserstoff, Cyclopropyl oder geradkettiges oder verzweigtes Alkyl mit bis zu 4 Kohlenstoffatomen bedeuten, |

und deren Salze.

Das Verfahren zur Herstellung der erfindungsgemäßen Verbindungen der allgemeinen Formel (I) ist dadurch gekennzeichnet,

daß man im Fall, daß $R^1$ und $R^2$ die oben angegebene Bedeutung haben, aber nicht gemeinsam einen Lactonring bilden,

[A] Verbindungen der allgemeinen Formel (II)

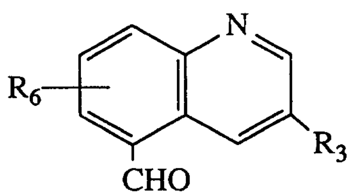 (II)

in welcher

$R^3$ und $R^6$ die oben angegebene Bedeutung haben,

zunächst mit Acylderivaten der allgemeinen Formel (III)

$R^5$-CO-$CH_2$-$R^4$ (III)

in welcher

$R^4$ und $R^5$ die oben angegebene Bedeutung haben,

gegebenenfalls unter Isolierung der entsprechenden Ylidenverbindungen der allgemeinen Formel (IV)

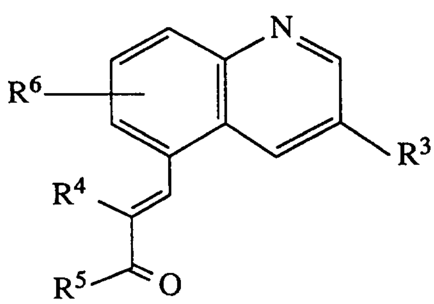 (IV)

in welcher

$R^3$, $R^4$, $R^5$ und $R^6$ die oben angegebene Bedeutung haben,

umsetzt und anschließend entweder mit Verbindungen der Formel (V)

$R^1$-CO-$CH_2$-$R^2$ (V)

in welcher

$R^1$ und $R^2$ die oben angegebene Bedeutung haben,

in Anwesenheit von Ammoniak oder Ammoniumsalzen, oder direkt mit Aminoderivaten der allgemeinen Formel (VI)

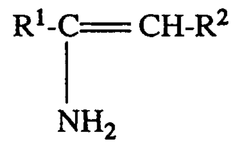

(VI)

in welcher

$R^1$ und $R^2$ die oben angegebene Bedeutung haben,

gegebenenfalls in Gegenwart von inerten organischen Lösemitteln umsetzt,

oder

[B] die Aldehyde der allgemeinen Formel (II) zunächst mit den Verbindungen der allgemeinen Formel (V), gegebenenfalls unter Isolierung der Ylidenverbindungen der allgemeinen Formel (VII)

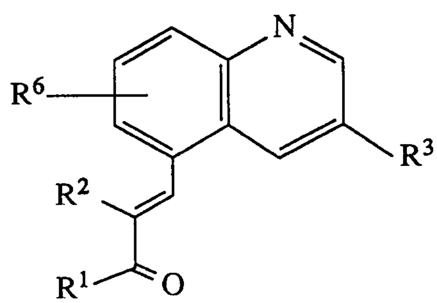

(VII)

in welcher

$R^1$, $R^2$, $R^3$ und $R^6$ die oben angegebene Bedeutung haben,

umsetzt und in einem nächsten Schritt mit den oben aufgeführten Verbindungen der allgemeinen Formel (III) in inerten Lösemitteln, in Anwesenheit von Ammoniak oder Ammoniumsalzen oder direkt mit Enaminosäurederivaten der allgemeinen Formel (VIII)

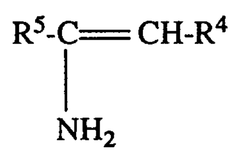

(VIII)

in welcher

$R^4$ und $R^5$ die oben angegebene Bedeutung haben,

umsetzt,

oder im Fall, daß $R^1$ und $R^2$ gemeinsam einen Lactonring bilden,

[C] zunächst nach denen unter [A] und [B] aufgeführten Methoden, Verbindungen der allgemeinen Formel (IX)

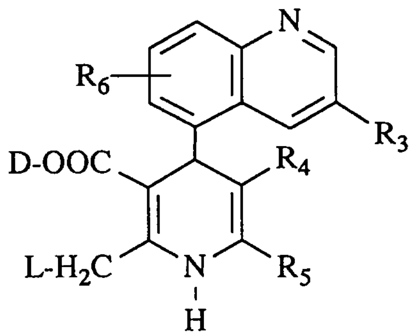

(IX)

in welcher

$R^3$, $R^4$, $R^5$ und $R^6$ die oben angegebene Bedeutung haben,

D für einen $C_1$-$C_6$-Alkyl-Rest steht

und

L für eine Abgangsgruppe wie beispielsweise Chlor oder Acetoxy steht,

herstellt und nach bekannten Verfahren einen säure- oder basenkatalysierten Ringschluß anschließt,

oder

[D] im Fall, daß $R^4$ für den Rest der Formel -CO-A-$R^8$ (A = Sauerstoff) oder -CO-$NR^9R^{10}$ steht, Verbindungen der allgemeinen Formel (X)

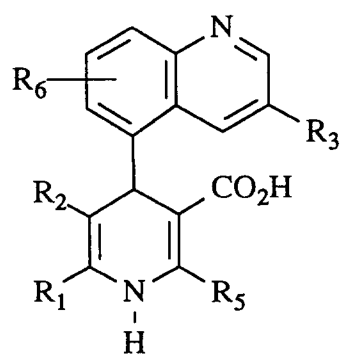

(X)

in welcher

$R^1$, $R^2$, $R^3$, $R^5$,und $R^6$ die oben angegebene Bedeutung haben,

gegebenenfalls über ein reaktives Säurederivat mit Alkoholen oder Aminen der allgemeinen Formel (XI) oder (XII)

HA - $R^8$ (XI)

oder

$HNR^9R^{10}$ (XII)

in welcher

A, $R^8$, $R^9$ und $R^{10}$ die oben angegebene Bedeutung haben,

umsetzt, wobei bei Einsatz chiraler Carbonsäuren die entsprechenden chiralen Ester oder Amide erhalten werden.

Die erfindungsgemäßen Verfahren können durch folgendes Formelschema erläutert werden:

[A]

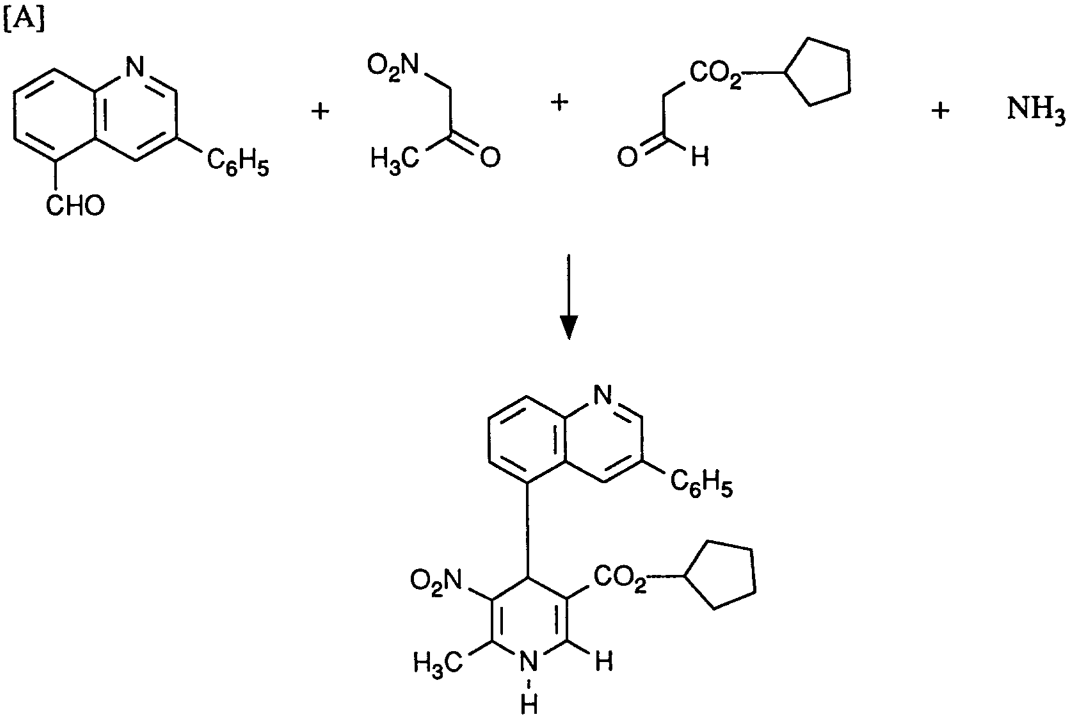

[B]

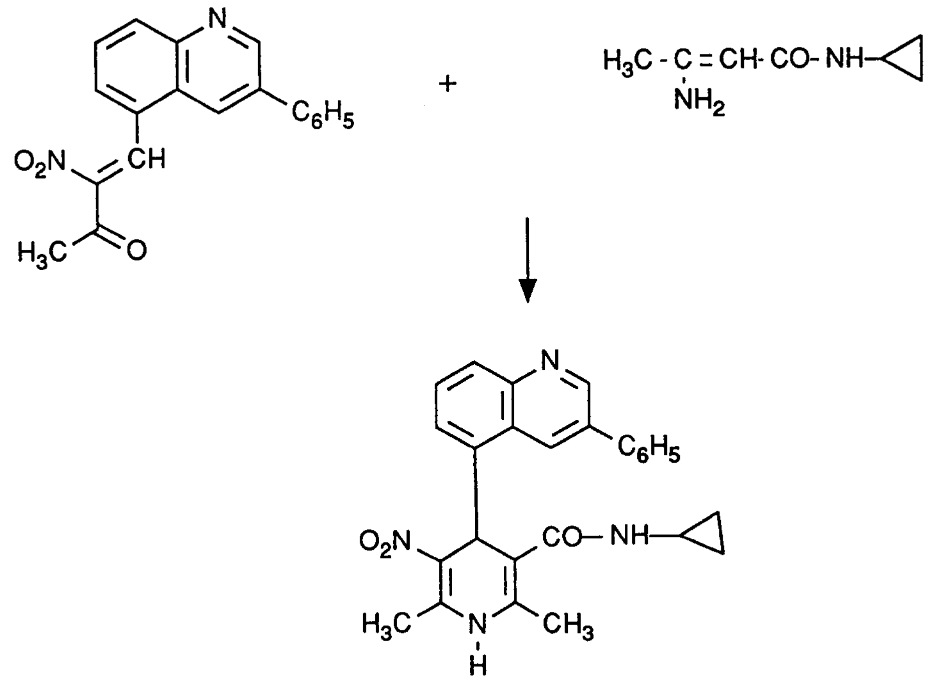

[C]

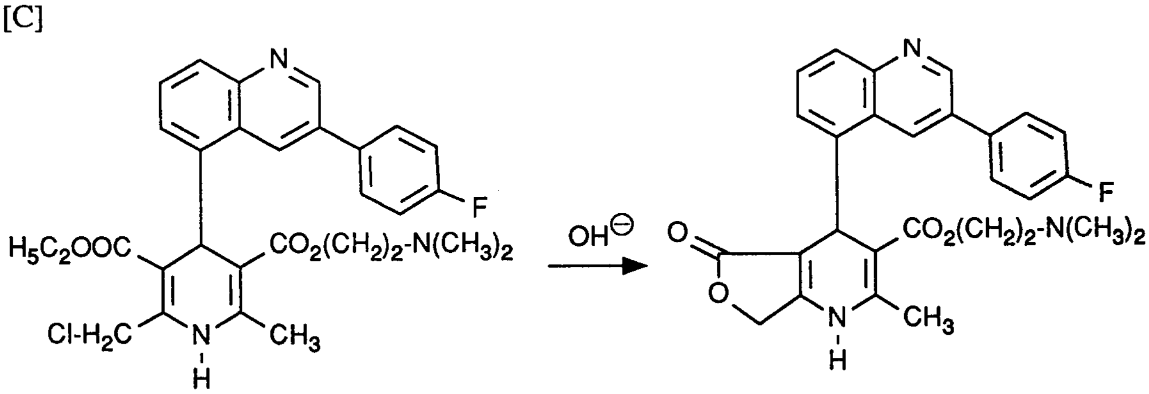


[D]

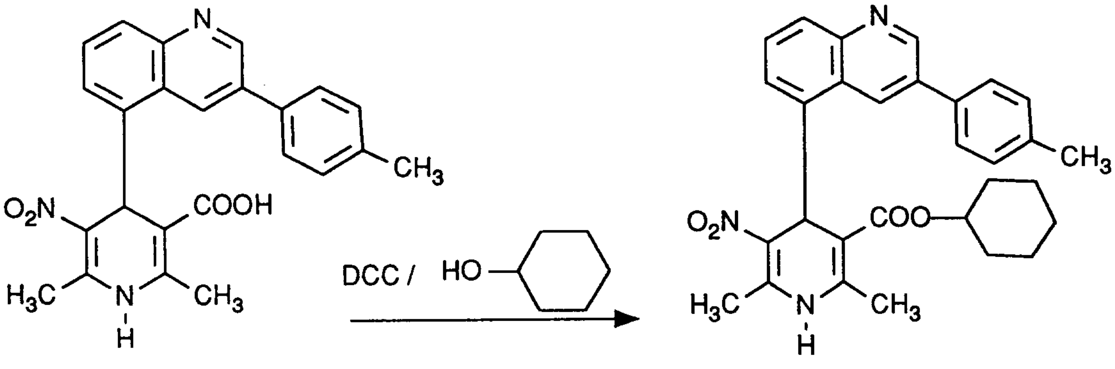


Als Lösemittel eignen sich für die Verfahren [A], [B] und [C] alle inerten organischen Lösemittel. Hierzu gehören bevorzugt Alkohole wie Methanol, Ethanol, n-bzw. iso-Propanol, Ether wie Diethylether, Tetrahydrofuran, Dioxan oder Glykolmono- oder dimethylether, Eisessig, Pyridin, Dimethylformamid, Dimethylsulfoxid,

Acetonitril oder Hexamethylphosphorsäuretriamid oder Toluol.

Als Lösemittel für das Verfahren [D] eignen sich die oben aufgeführten Lösemittel mit Ausnahme der Alkohole.

Die Reaktionstemperaturen für die Verfahren [A], [B], [C] und [D] können in einem größeren Bereich variiert werden. Im allgemeinen arbeitet man in einem Bereich von 10 °C bis 200 °C, bevorzugt von 20 °C bis 150 °C.

Die Verfahren können bei Normaldruck, erhöhtem oder erniedrigtem Druck (beispielsweise von 0,5 bis 5 bar), vorzugsweise bei Normaldruck durchgeführt werden.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Enantiomerenreine Formen erhält man z.B., indem man Diastereomerengemische der Verbindungen der allgemeinen Formel (I), in welcher $R^4$ für einen optischen Esterrest steht, nach üblicher Methode trennt, anschließend die enantiomerenreinen Carbonsäuren herstellt und dann beispielsweise durch Veresterung mit entsprechenden Alkoholen in die enantiomerenreinen Dihydropyridincarbonsäureester überführt.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Veresterung der enantiomerenreinen Dihydropyridine erfolgt bevorzugt in Ethern wie Diethylether oder Tetrahydrofuran, Dimethylformamid, Methylenchlorid, Chloroform, Acetonitril oder Toluol.

Die Aldehyde der allgemeinen Formel (II) sind bekannt oder können nach üblichen Methoden hergestellt werden (US 5 100 900).

Auch die Acylderivate der Formel (III), die Ylidenverbindungen (IV) und (VII), die Enaminoderivate der Formel (VI) und (VIII) sind bekannt oder können nach üblichen Methoden hergestellt werden.

Die vorstehenden Herstellungsverfahren sind lediglich zur Verdeutlichung angegeben. Die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Insbesondere wirken sie positiv inotrop.

Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32 °C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert. Der Perfusionsdruck wird mittels eines Druckaufnehmers, der vor dem Herzen mit dem Perfusionssystem in Verbindung steht, registiert. Unter diesen Bedingungen zeigt eine Senkung des Perfusionsdrucks eine Koronardilatation, eine Zu- bzw.

Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Beispiel 1

2-Methyl-4-[3-(4-methylphenyl)-chinolin-5-yl]-5-oxo-1,4,5,7-tetrahydrofuro[3,4-b]pyridin-3-carbonsäure-2-(N-benzyl-N-methylamino)ethylester

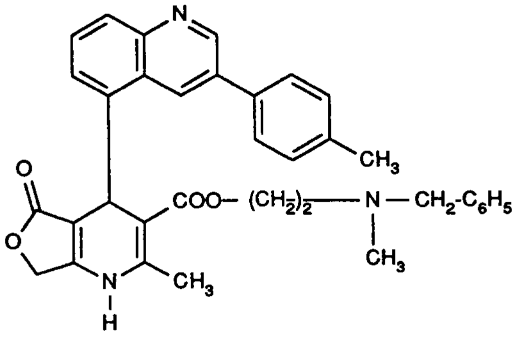

2,3 g (5,5 mmol) 2-[3-(4-Methylphenyl)-chinolin-5-yliden)-4-acetoxy-3-oxo-buttersäureethylester werden in 30 ml Isopropanol mit 1,24 g (5 mmol) 3-Aminocrotonsäure-2-(N-benzyl-4-methylamino)-ethylester versetzt und 24 Stunden zum Rückfluß erhitzt. Es wird abgekühlt, eingeengt und grob durch Flash-Chromatographie gereinigt, wobei man 2,3 g eines vorgereinigten Öls erhält. Dieses Öl wird in einer Mischung von 1 g Kaliumhydroxid in 40 ml Isopropanol 30 Minuten zum Rückfluß erhitzt. Es wird abgekühlt, mit 1 N Salzsäure neutral gestellt, eingeengt und in Essigester/Wasser aufgenommen. Es wird getrennt, die Essigesterphase wird mit Wasser gewaschen, getrocknet und eingeengt. Das erhaltene Rohprodukt wird über eine Kieselgelsäule mit Toluol/Essigester-Gemischen gereinigt. Die sauberen Fraktionen werden gesammelt, eingeengt und mit Methanol kristallisiert. Man erhält 550 mg farblose Kristalle vom Schmelzpunkt 173-175 °C.

Beispiel 2

2,6-Dimethyl-3-nitro-4-(3-phenylchinolin-5-yl)-1,4-dihydropyridin-5-carbonsäure-cyclopropylamid

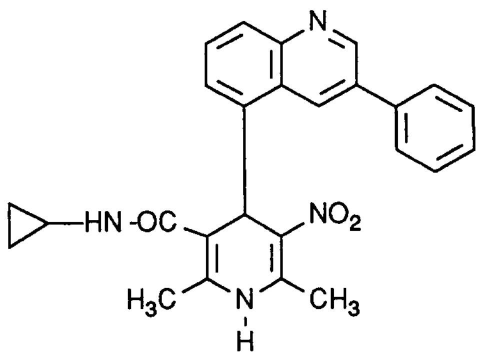

2,33 g (10 mmol) 3-Phenylchinolin-5-carbaldehyd werden in 40 ml Ethanol mit 1,4 g (10 mmol) 3-Aminocrotonsäure-cyclopropylamid, 1,8 g (17,5 mmol) Nitroaceton und 0,6 ml (10 mmol) Essigsäure versetzt und 4 Stunden zum Rückfluß erhitzt. Es wird abgekühlt, eingeengt und in Essigester gelöst. Die Essigesterlösung wird mit Wasser, Natriumhydrogencarbonatlösung und wieder Wasser gewaschen, getrocknet und eingeengt. Es wird über eine Kieselgelsäule mit Essigester/Toluol-Gemischen gereinigt. Die reinen Fraktionen werden vereint, eingeengt und mit Ethanol kristallisiert. Man erhält 420 mg gelbe Kristalle vom Schmelzpunkt 179-182 °C.

In Analogie zu den Vorschriften der Beispiele 1 und 2 werden die in den Tabellen 1 und 2 aufgeführten Verbindungen hergestellt:

Tabelle 1:

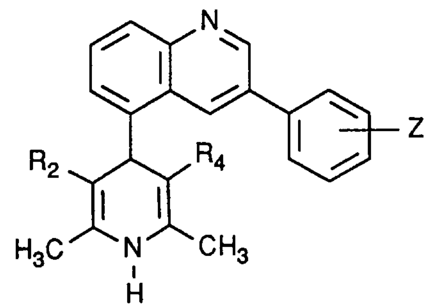

| Bsp.-Nr. | $R_2$ | $R_4$ | Z | F°C | Enantiomer |
|---|---|---|---|---|---|
| 3 | -CN | $-CO_2-(CH_2)_5-NH_2$ | H | 138 | |
| 4 | -CN | $-CO-CH_3$ | H | 244-249 | |
| 5 | -CN | $-CO_2-(CH_2)_2-NH_2$ | H | 217 (Zers.) | |
| 6 | -CN | 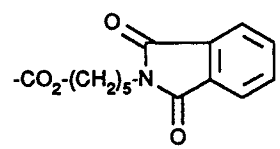 | H | 118 | |
| 7 | -CN | 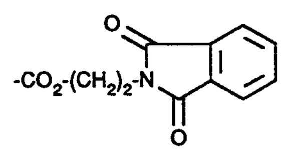 | H | 168 | |
| 8 | -CN | 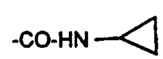 | H | 202 | |

Tabelle 1:

| Bsp.-Nr. | $R^2$ | $R^4$ | Z | F°C | Enantiomer |
|---|---|---|---|---|---|
| 9 | -CN | 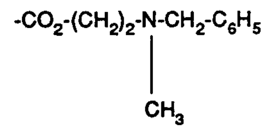 | p-F | 186 | |
| 10 | -CN | 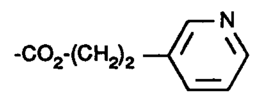 | o-Cl | 199 | |
| 11 | -CN | 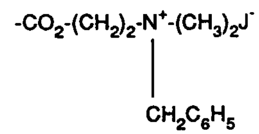 | H | Schaum | |
| 12 | -CN | 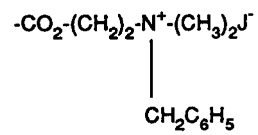 | H | Schaum | |
| 13 | -CN | 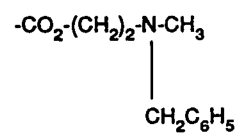 | o-F | 157 | |
| 14 | -CN | 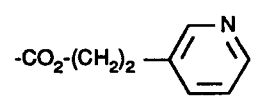 | H | 159 | |

Tabelle 1:

| Bsp.-Nr. | $R^2$ | $R^4$ | Z | F°C | Enantiomer |
|---|---|---|---|---|---|
| 15 | -CN | $-CO_2-(CH_2)_2-N-CH_3$<br>$CH_2C_6H_5$ | H | Schaum | (+) |
| 16 | -CN | $-CO_2-(CH_2)_2-N-CH_3$<br>$CH_2C_6H_5$ | H | Schaum | |
| 17 | -CN | $-CO_2-(CH_2)_2-N-CH_3$<br>$CH_2C_6H_5$ | H | 195 | |
| 18 | $-NO_2$ | $-CO-CH_3$ | H | 284 | |
| 19 | $-NO_2$ | $-CO_2-(CH_2)_2-N-CH_3$<br>$CH_2C_6H_5$ | H | 220 | |
| 20 | CN | 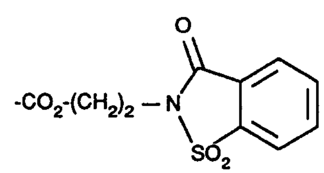 | H | 167-168 | |

Tabelle 2:

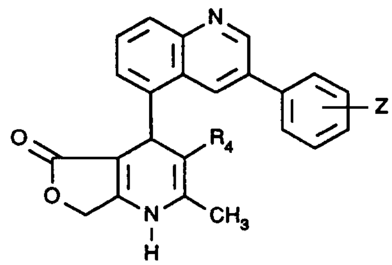

| Bsp.-Nr. | $R^4$ | Z | F°C | Enantiomer |
|---|---|---|---|---|
| 21 | $-CONH-CH_3$ | H | | |
| 22 | $-CO-NH-C_2H_5$ | H | 190 | |
| 23 | 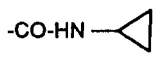 | H | 202-204 | |
| 24 | 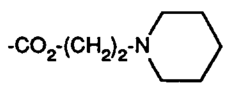 | H | 210-212 | |
| 25 | $-CO_2-(CH_2)_2-N(CH_3)_2$ | H | 239-240 | |
| 26 | 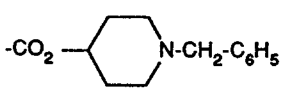 | p-$CH_3$ | 160 (Zers.) | |
| 27 | $-CO_2-(CH_2)_2-N-CH_3$<br>$CH_2C_6H_5$ | H | 176 | |
| 28 | 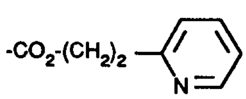 | H | 172 | |
| 29 | 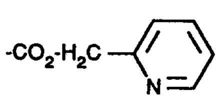 | H | 262 | |

Tabelle 2:

| Bso.-Nr. | $R^4$ | Z | F°C | Enantiomer |
|---|---|---|---|---|
| 30 | 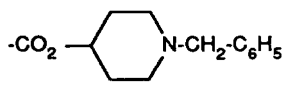 | H | 223-230 | |
| 31 | $-CO_2-(CH_2)_2-N(CH_3)-CH_2-C_6H_5$ | m-$OCH_3$ | 169-172 | |
| 32 | 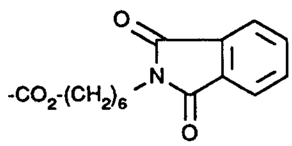 | H | 235 (Zers.) | |
| 33 | 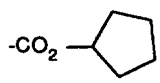 | p-F | >280 | |
| 34 | $-CO_2-(CH_2)_2-N(CH_3)-CH_2-C_6H_5$ | p-F | 169-170 | |
| 35 | 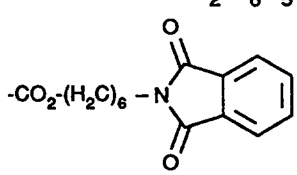 | m-$OCH_3$ | Schaum | |
| 36 | $-COO-(CH_2)_2-NH-COO-CH_2-C_6H_5$ | H | 142-144 | |
| 37 | 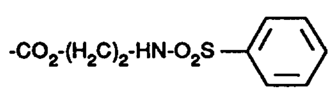 | H | 214-216 | |

**Patentansprüche**

**1.** 4-Chinolyl-dihydropyridinester der allgemeinen Formel (I)

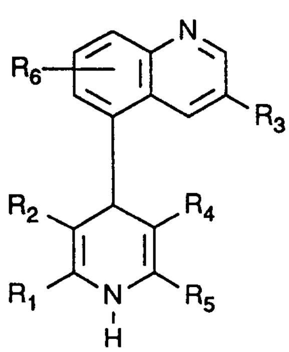

(I)

in welcher

$R^1$ und $R^5$ gleich oder verschieden sind und für geradkettiges oder verzweigtes Alkyl mit bis zu 8 Kohlenstoffatomen stehen,

$R^2$ für Nitro oder Cyano steht,

oder

$R^1$ und $R^2$ gemeinsam einen Lactonring der Formel

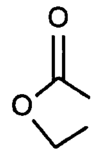

bilden,

worin

$R^6$ für Wasserstoff, Halogen oder geradkettiges oder verzweigtes Alkyl oder Alkoxy mit jeweils bis zu 8 Kohlenstoffatomen steht,

$R^3$ für Aryl mit 6 bis 10 Kohlenstoffatomen steht, das gegebenenfalls bis zu 2-fach gleich oder verschieden durch Halogen, Nitro, Cyano, Hydroxy, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder für Thienyl oder Pyridyl steht, die gegebenenfalls durch Halogen substituiert sind,

$R^4$ für Cyano, Nitro oder Formyl steht

oder

$R^4$ und $R^5$ gemeinsam einen Lactonring der Formel

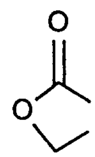

bilden,

oder

$R^4$ für eine Gruppe der Formel $-CO-A-R^8$ oder $-CO-NR^9R^{10}$ steht,

worin

A eine direkte Bindung oder ein Sauerstoffatom bedeutet,

$R^8$ Cycloalkyl mit 3 bis 8 Kohlenstoffatomen bedeutet, das gegebenenfalls durch die Gruppe $-NR^{11}$ unterbrochen ist, oder einen geradkettigen, verzweigten, cyclischen, gesättigten oder ungesättigten Kohlenwasserstoffrest mit bis zu 12 Kohlenstoffatomen bedeutet, der gegebenenfalls durch Sauerstoff,

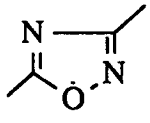

oder durch Aryliden mit 6 bis 10 Kohlenstoffatomen unterbrochen ist, wobei das Aryliden gegebenenfalls durch Halogen, Nitro, Amino, Hydroxy, Carboxy oder durch geradkettiges oder verzweigtes Alkyl, Alkoxy oder Alkoxycarbonyl mit jeweils bis zu 8 Kohlenstoffatomen substituiert ist oder der Kohlenwasserstoffrest gegebenenfalls durch Phenyl substituiert oder ersetzt ist, das seinerseits durch Fluor, Chlor, Methyl oder Methoxy substituiert sein kann, oder der Kohlenwasserstoffrest gegebenenfalls durch eine Gruppe der Formel $-S(O)_a$ oder $-NR^{11}$ unterbrochen ist,

a eine Zahl 0, 1 oder 2 bedeutet

und

$R^{11}$ Wasserstoff oder Aryl mit 6 bis 10 Kohlenstoffatomen bedeutet, das gegebenenfalls durch Halogen, Nitro, Phenyl oder geradkettiges oder verzweigtes Alkyl oder Alkoxy mit jeweils bis zu 4 Kohlenstoffatomen substituiert ist,
oder geradkettiges oder verzweigtes Alkyl mit bis zu 6 Kohlenstoffatomen oder Cycloalkyl mit 3 bis 8 Kohlenstoffatomen bedeutet, wobei sowohl Alkyl als auch Cycloalkyl gegebenenfalls durch Aryl mit 6 bis 10 Kohlenstoffatomen substituiert sind
und wobei der Kohlenwasserstoffrest im Falle, daß A für Sauerstoff steht, immer ein- oder zweifach gleich oder verschieden substituiert ist durch Cycloalkyl mit 3 bis 8 Kohlenstoffatomen oder durch $-CO-NR^{12}R^{13}$, $-NR^{14}-CO-R^{15}$, $-NR^{16}-SO_2-R^{17}$, $-SO_2-NR^{18}R^{19}$, $O-NO_2$, $-O-(CH_2)_b-R^{20}$, $-S(O)_c-(CH_2)_d-R^{21}$, $-NR^{22}R^{23}$ oder $-NR^{24}COOR^{25}$,

worin

$R^{12}$, $R^{13}$, $R^{14}$, $R^{15}$, $R^{16}$, $R^{17}$, $R^{18}$, $R^{19}$, $R^{24}$ und $R^{25}$ gleich oder verschieden sind und die oben angegebene Bedeutung von $R^{11}$ haben und mit dieser gleich oder verschieden sind,

b eine Zahl 1, 2, 3, 4 oder 5 bedeutet,

d eine Zahl 0, 1, 2, 3,4 oder 5 bedeutet,

c die oben angegebene Bedeutung von a hat und mit dieser gleich oder verschieden ist,

$R^{20}$ und $R^{21}$ gleich oder verschieden sind und Aryl mit 6 bis 10 Kohlenstoffatomen bedeuten, das gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert ist,

$R^{22}$ und $R^{23}$ gleich oder verschieden sind und Wasserstoff oder einen geradkettigen, verzweigten, gesättigen, ungesättigen oder cyclischen Kohlenwasserstoffrest mit bis zu 8 Kohlenstoffatomen bedeuten, der gegebenenfalls bis zu 2-fach gleich oder verschieden durch Hydroxy, Halogen, Cycloalkyl mit 3 bis 6 Kohlenstoffatomen oder durch Aryl oder Aryloxy mit 6 bis 10 Kohlenstoffatomen substituiert ist, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können,
oder
Aryl mit 6 bis 10 Kohlenstoffatomen bedeuten, das gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert ist,

oder

$R^{22}$ und $R^{23}$ gemeinsam mit dem Stickstoffatom einen 5- bis 7-gliedrigen, gesättigen oder ungesättigten Heterocyclus mit bis zu 3 Heteroatomen aus der Reihe S, N oder O bilden, der gegebenenfalls durch geradkettiges oder verzweigtes Alkyl mit bis zu 4 Kohlenstoffatomen substituiert ist, das seinerseits durch Phenyl substituiert sein kann, oder gegebenenfalls durch Phenyl substituiert ist, das seinerseits durch Halogen substituiert sein kann,
oder der Kohlenwasserstoffrest im Falle, daß A Für Sauerstoff steht, substituiert ist durch einen 3- bis 7-gliedrigen gesättigten oder ungesättigten Heterocyclus oder

Heterocyclyloxyring, der bis zu 3 Heteroatome aus der Reihe S, N oder O oder Gruppen der Formel CO oder $SO_2$ enthalten kann,
wobei der Heterocyclus seinerseits bis zu 2-fach gleich oder verschieden durch Halogen, Hydroxy oder durch Aryl oder Arylsulfonyl mit 6 bis 10 Kohlenstoffatomen substituiert sein kann, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können,
oder der Heterocyclus gegebenenfalls bis zu 2-fach gleich oder verschieden durch geradkettiges oder verzweigtes Alkyl, Alkoxy oder Alkenyl mit jeweils bis zu 8 Kohlenstoffatomen substituiert ist, die ihrerseits bis zu 2-fach gleich oder verschieden durch - $NR^{26}R^{27}$ substituiert sein können,

worin

$R^{26}$ und $R^{27}$ die oben angegebene Bedeutung von $R^{22}$ und $R^{23}$ haben und mit diesen gleich oder verschieden sind, und die gesamten Alkyl- und Alkenyl-Reste gegebenenfalls ihrerseits durch Phenyl oder Phenoxy substituiert sind, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 4 Kohlenstoffatomen substituiert sein können,

$R^9$ und $R^{10}$ gleich oder verschieden sind und Wasserstoff, einen geradkettigen, verzweigten, cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,

oder

$R^9$ und $R^{10}$ gemeinsam unter Einbezug des Stickstoffatoms einen 3-bis 8-gliedrigen, gesättigen oder ungesättigten Heterocyclus bilden, der gegebenenfalls durch Sauerstoff oder durch einen Rest der Formel $S(O)_e$,-CO-oder -$NR^{28}$ unterbrochen sein kann,

worin

e eine Zahl 0, 1 oder 2 bedeutet,

$R^{28}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist, oder
einen cyclischen, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit 3 Heteroatomen aus der Reihe S, N oder O substituiert ist, die ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, und der gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert ist,

und deren Salze.

2. Verbindungen der allgemeinen Formel (I) gemäß Anspruch 1, in welcher
   $R^1$ und $R^5$ gleich oder verschieden sind und für geradkettiges oder verzweigtes Alkyl mit bis zu 4 Kohlenstoffatomen stehen,
   $R^2$ für Nitro oder Cyano steht,
   oder
   $R^1$ und $R^2$ gemeinsam einen Lactonring der Formel

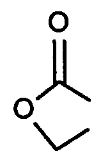

bilden,
   $R^6$ für Wasserstoff, Fluor oder Chlor steht,
   $R^3$ für Phenyl steht, das gegebenenfalls bis zu zweifach durch Fluor, Chlor, Brom, Nitro, Cyano, Hydroxy, Trifluormethyl oder durch geradkettiges oder verzweigtes Alkyl, Alkoxy oder Alkoxycarbonyl mit jeweils bis zu 4 Kohlenstoffatomen substituiert ist, oder für
   Thienyl oder Pyridyl steht, die gegebenenfalls durch Fluor, Chlor oder Brom substituiert sind,
   $R^4$ für Cyano, Nitro oder Formyl steht
   oder
   $R^4$ und $R^5$ gemeinsam einen Lactonring der Formel

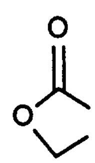

bilden,
   oder
   $R^4$ für eine Gruppe der Formel -CO-A-$R^8$ oder -CO-$NR^9R^{10}$ steht,
   worin
   A eine direkte Bindung oder ein Sauerstoffatom bedeutet,
   $R^8$ Cyclopropyl, Cyclobutyl, Cyclopentyl, Cyclohexyl oder Cycloheptyl bedeutet, die gegebenenfalls durch die Gruppe -$NR^{11}$ unterbrochen sind, oder
   einen geradkettigen, verzweigten, cyclischen, gesättigten oder ungesättigten Kohlenwasserstoffrest mit bis zu 10 Kohlenstoffatomen bedeutet, der gegebenenfalls durch Phenyl substituiert ist, und der gegebenenfalls durch Sauerstoff, Phenyliden oder durch eine Gruppe der Formel

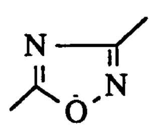 ,

-$S(O)_a$ oder -$NR^{11}$ unterbrochen ist,
   worin
   a eine Zahl 0, 1 oder 2 bedeutet
   und
   $R^{11}$ Wasserstoff oder Phenyl bedeutet, das gegebenenfalls durch Halogen, Methyl, Methoxy oder Nitro substituiert ist, oder Cyclopropyl, Cyclopentyl, Cyclohexyl oder geradkettiges oder verzweigtes Alkyl mit bis zu 5 Kohlenstoffatomen bedeutet, das gegebenenfalls durch Phenyl

substituiert ist,

und wobei der Kohlenwasserstoffrest im Falle, daß A für Sauerstoff steht, immer substituiert ist durch Cyclopropyl, Cyclobutyl, Cyclopentyl, Cyclohexyl oder durch eine Gruppe $-CO-NR^{12}R^{13}$, $-NR^{14}-CO-R^{15}$, $-NR^{16}-SO_2-R^{17}$, $-SO_2-NR^{18}R^{19}$, $O-NO_2$, $-O-(CH_2)_b-R^{20}$, $-S(O)_c-(CH_2)_d-R^{21}$, $-NR^{22}R^{23}$ oder $-NR^{24}-COOR^{25}$,

worin

| | |
|---|---|
| $R^{12}$, $R^{13}$, $R^{14}$, $R^{15}$, $R^{16}$, $R^{17}$, $R^{18}$, $R^{19}$, $R^{24}$ und $R^{25}$ | gleich oder verschieden sind und die oben angegebene Bedeutung von $R^{11}$ haben und mit dieser gleich oder verschieden sind, |
| b | eine Zahl 1, 2, 3 oder 4 bedeutet, |
| d | eine Zahl 0, 1, 2, 3 oder 4 bedeutet, |
| c | die oben angegebene Bedeutung von a hat und mit dieser gleich oder verschieden ist, |
| $R^{20}$ und $R^{21}$ | gleich oder verschieden sind und Phenyl bedeuten, das gegebenenfalls bis zu 2-fach gleich oder verschieden durch Fluor, Chlor, Brom, Nitro, Hydroxy, Carboxy oder durch geradkettiges oder verzweigtes Alkyl, Alkoxy oder Alkoxycarbonyl mit jeweils bis zu 4 Kohlenstoffatomen substituiert ist, |
| $R^{22}$ und $R^{23}$ | gleich oder verschieden sind und Wasserstoff oder einen geradkettigen, verzweigten, gesättigen, ungesättigen oder cyclischen Kohlenwasserstoffrest mit bis zu 6 Kohlenstoffatomen bedeuten, der gegebenenfalls bis zu 2-fach gleich oder verschieden durch Fluor, Chlor, Cyclopropyl, Cyclopentyl, Cyclohexyl, Phenyl oder Phenoxy substituiert ist, die ihrerseits durch Fluor, Chlor, Brom, Nitro, Amino, Hydroxy, Carboxy oder durch geradkettiges oder verzweigtes Alkyl, Alkoxy oder Alkoxycarbonyl mit jeweils bis zu 4 Kohlenstoffatomen substituiert sein können, oder Phenyl bedeuten, das gegebenenfalls durch Fluor, Chlor, Hydroxy, Carboxy oder durch geradkettiges oder verzweigtes Alkyl, Alkoxy oder Alkoxycarbonyl mit jeweils bis zu 3 Kohlenstoffatomen substituiert ist, |

oder

$R^{22}$ und $R^{23}$ gemeinsam mit dem Stickstoffatom einen Morpholin-, Piperidin- oder Piperazinring bilden, die gegebenenfalls durch geradkettiges oder verzweigtes Alkyl mit bis zu 4 Kohlenstoffatomen, Phenyl oder Benzyl substituiert sind,

oder der Kohlenwasserstoffrest im Falle, daß A für Sauerstoff steht, substituiert ist durch Pyridyl, Tetrahydropyranyl, Pyrazolyl, Furyl, Chromanyl, Piperazinyl, Piperidinyl, Tetrahydroisochinolidinyl oder durch einen Rest der Formel

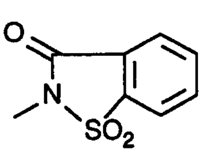 , 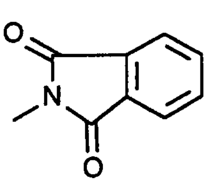 oder 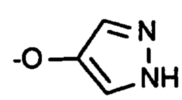

wobei die Heterocyclen ihrerseits durch Fluor, Chlor, geradkettiges oder verzweigtes Alkyl oder Alkoxy mit jeweils bis zu 3 Kohlenstoffatomen oder durch Phenyl, Benzyl oder Phenylsulfonyl substituiert sein können, die ihrerseits durch Fluor, Chlor, Brom, Nitro, Amino, Hydroxy, Carboxy oder durch geradkettiges oder verzweigtes Alkyl, Alkoxy oder Alkoxycarbonyl mit jeweils bis zu 3 Kohlenstoffatomen substituiert sein

können,

$R^9$ und $R^{10}$ gleich oder verschieden sind und Wasserstoff oder einen geradkettigen, verzweigten, cyclischen, gesättigten oder ungesättigten Kohlenwasserstoffrest mit bis zu 6 Kohlenstoffatomen bedeuten, der gegebenenfalls durch Fluor, Chlor oder Phenyl substituiert ist, oder

Phenyl bedeuten, das gegebenenfalls durch Fluor, Chlor, geradkettiges oder verzweigtes Alkyl, Alkoxy, Alkylthio, Alkoxycarbonyl mit jeweils bis zu 6 Kohlenstoffatomen, Trifluormethyl oder Trifluormethoxy substituiert ist,

oder

$R^9$ und $R^{10}$ gemeinsam unter Einbezug des Stickstoffatoms einen 5-bis 6-gliedrigen, gesättigen oder ungesättigten Heterocyclus bilden, der gegebenenfalls durch ein Sauerstoffatom oder durch einen Rest der Formel $-NR^{28}$ unterbrochen sein kann,

worin

$R^{28}$ Wasserstoff, Phenyl oder einen cyclischen, geradkettigen oder verzweigten Kohlenwasserstoffrest mit bis zu 6 Kohlenstoffatomen bedeutet,

und deren Salze.

**3.** Verbindungen der allgemeinen Formel (I) gemäß Anspruch 1, in welcher

$R^1$ und $R^5$ gleich oder verschieden sind und für Methyl oder Ethyl stehen,

$R^2$ für Nitro oder Cyano steht,

oder

$R^1$ und $R^2$ gemeinsam einen Lactonring der Formel

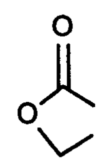

bilden,

$R^6$ für Wasserstoff steht,

$R^3$ für Phenyl steht, das gegebenenfalls durch Fluor, Chlor, Nitro, Cyano, Hydroxy, Trifluormethyl, Methyl, Ethyl, Methoxy oder Ethoxy substituiert ist,

$R^4$ für Cyano, Nitro oder Formyl steht

oder

$R^4$ und $R^5$ gemeinsam einen Lactonring der Formel

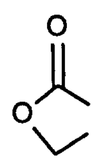

bilden,

oder

$R^4$ für eine Gruppe der Formel $-CO-A-R^8$ oder $-CO-NR^9R^{10}$ steht,

worin

A eine direkte Bindung oder ein Sauerstoffatom bedeutet,

$R^8$ Cyclopropyl, Cyclobutyl, Cyclopentyl oder Cyclohexyl bedeutet, die gegebenenfalls durch die Gruppe $-NR^{11}$ unterbrochen sind, oder

einen geradkettigen, verzweigten, cyclischen, gesättigten oder ungesättigten substituierten Kohlenwasserstoffrest mit bis zu 8 Kohlenstoffatomen bedeutet, der gegebenenfalls durch ein Sauerstoffatom, Phenyliden oder durch eine Gruppe der Formel $-S(O)_a$ oder $-NR^{11}$ unterbrochen ist und der gegebenenfalls durch Phenyl substituiert ist,

worin

a eine Zahl 0 oder 2 bedeutet

und

$R^{11}$ Wasserstoff oder Phenyl bedeutet, das gegebenenfalls durch Fluor, Chlor, Methyl oder Methoxy substituiert ist, oder
Cyclopropyl, Cyclopentyl, Cyclohexyl, oder geradkettiges oder verzweigtes Alkyl mit bis zu 4 Kohlenstoffatomen bedeutet, das gegebenenfalls durch Phenyl substituiert ist
und wobei der Kohlenwasserstoffrest im Falle, daß A für Sauerstoff steht, immer substituiert ist durch Cyclopropyl, Cyclobutyl, Cyclopentyl, Cyclohexyl oder durch eine Gruppe $-CO-NR^{12}R^{13}$, $-NR^{14}-CO-R^{15}$, $-NR^{16}-SO_2-R^{17}$, $-SO_2-NR^{18}R^{19}$, $O-NO_2$, $-O-(CH_2)_b-R^{20}$, $-S(O)_c-(CH_2)_d-R^{21}$, $-NR^{22}R^{23}$ oder $-NR^{24}-COOR^{25}$,

worin

$R^{12}$, $R^{13}$, $R^{14}$, $R^{15}$, $R^{16}$, $R^{17}$, $R^{18}$, $R^{19}$, $R^{24}$ und $R^{25}$ gleich oder verschieden sind und die oben angegebene Bedeutung von $R^{11}$ haben und mit dieser gleich oder verschieden sind,

b eine Zahl 1, 2, 3 oder 4 bedeutet,

d eine Zahl 0, 1, 2, 3 oder 4 bedeutet,

c die oben angegebene Bedeutung von a hat und mit dieser gleich oder verschieden ist,

$R^{20}$ und $R^{21}$ gleich oder verschieden sind und Phenyl bedeuten, das gegebenenfalls durch Fluor, Chlor, Methyl oder Methoxy substituiert ist,

$R^{22}$ und $R^{23}$ gleich oder verschieden sind und Wasserstoff oder einen geradkettigen, verzweigten, gesättigen, ungesättigen oder cyclischen Kohlenwasserstoffrest mit bis zu 4 Kohlenstoffatomen bedeuten, der gegebenenfalls durch Fluor, Chlor, Cyclopropyl, Cyclopentyl, Cyclohexyl, Phenyl oder Phenoxy substituiert ist, die ihrerseits durch Fluor, Chlor, Hydroxy oder durch Methyl oder Methoxy substituiert sein können, oder
Phenyl bedeuten, das gegebenenfalls durch Fluor, Chlor, Methyl oder Methoxy substituiert ist,

oder

$R^{22}$ und $R^{23}$ gemeinsam mit dem Stickstoffatom einen Piperidin-oder Piperazinring bilden, der gegebenenfalls durch Benzyl substituiert ist,
oder der Kohlenwasserstoffrest im Falle, daß A für Sauerstoff steht, substituiert ist durch Pyridyl, Tetrahydropyranyl, Pyrazolyl, Furyl, Chromanyl, Piperazinyl, Piperidinyl, Isochinolidinyl oder durch einen Rest der Formel

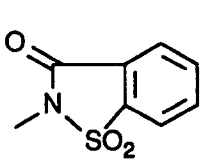 oder 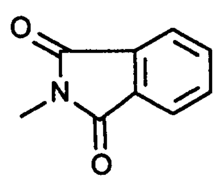

$R^9$ und $R^{10}$ gleich oder verschieden sind und Wasserstoff, einen geradkettigen, verzweigten, cyclischen, gesättigten oder ungesättigten Kohlenwasserstoffrest mit bis zu 6 Kohlenstoffatomen bedeuten, der gegebenenfalls durch Phenyl substituiert sein kann, oder
Phenyl oder Pyridyl bedeuten,

und deren Salze.

**4.** Verbindungen der allgemeinen Formel (I) gemäß Anspruch 1, in welcher

$R^1$ und $R^5$ für Methyl stehen,

$R^2$ für Cyano oder Nitro steht

oder

$R^1$ und $R^2$ gemeinsam einen Lactonring der Formel

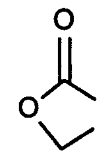

bilden,

$R^6$ für Wasserstoff steht,

und

$R^3$ für Phenyl steht, das gegebenenfalls durch Fluor, Chlor, Hydroxy, Methyl oder Methoxy substituiert ist,

$R^4$ für einen Rest der Formel $-CO-A-R^8$ oder $-CO-NR^9R^{10}$ steht,

worin

A eine direkte Bindung oder ein Sauerstoffatom bedeutet,

$R^8$ Cyclopropyl, Cyclobutyl, Cyclopentyl oder Cyclohexyl bedeutet, die gegebenenfalls durch die Gruppe $-NR^{11}$ unterbrochen sind, oder einen geradkettigen, verzweigten, cyclischen, gesättigten oder ungesättigten substituierten Kohlenwasserstoffrest mit bis zu 6 Kohlenstoffatomen bedeutet, der gegebenenfalls durch ein Sauerstoff-oder Schwefelatom oder die Gruppe $-NR^{11}$ unterbrochen ist,

worin

$R^{11}$ Methyl, Phenyl oder Benzyl bedeutet, und wobei der Kohlenwasserstoffrest im Falle, daß A für Sauerstoff steht, immer substituiert ist durch Pyridyl, Cyclopropyl, Cyclobutyl, Cyclopentyl, Cyclohexyl oder durch eine Gruppe $-NR^{14}-CO-R^{15}$, $-NR^{16}-SO_2-R^{17}$, $-O-(CH_2)_b-R^{20}$, $-NR^{22}R^{23}$, $-NR^{24}-COOR^{25}$,

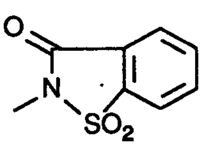

oder

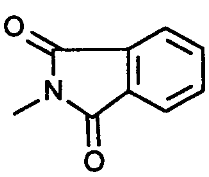

worin

$R^{14}$, $R^{15}$, $R^{16}$, $R^{17}$, $R^{24}$ und $R^{25}$ gleich oder verschieden sind und Wasserstoff oder Phenyl bedeuten, das gegebenenfalls durch Fluor, Chlor, Methyl oder Methoxy substituiert ist, oder geradkettiges oder verzweigtes Alkyl mit bis zu 4 Kohlenstoffatomen bedeuten, das gegebenenfalls durch Phenyl substituiert ist,

b eine Zahl 1 oder 2 bedeutet,

$R^{20}$ Phenyl bedeutet, das gegebenenfalls durch Methyl oder Methoxy substituiert ist,

$R^{22}$ und $R^{23}$ gleich oder verschieden sind und Wasserstoff, Phenyl oder einen geradkettigen oder verzweigten Kohlenwasserstoffrest mit bis zu 4 Kohlenstoffatomen bedeuten, der gegebenenfalls durch Cyclopropyl, Cyclopentyl Cyclohexyl, Phenyl oder Phenoxy substituiert ist, die ihrerseits durch Hydroxy, Methyl oder Methoxy substituiert sein können,

oder

$R^{22}$ und $R^{23}$ gemeinsam mit dem Stickstoffatom einen Piperidinring bilden, der gegebenenfalls durch Benzyl substituiert ist,

$R^9$ und $R^{10}$ gleich oder verschieden sind und Wasserstoff, Cyclopropyl oder geradkettiges oder verzweigtes Alkyl mit bis zu 4 Kohlenstoffatomen bedeuten,

und deren Salze.

**5.** Verfahren zur Herstellung von Verbindungen der allgemeinen Formel (I)

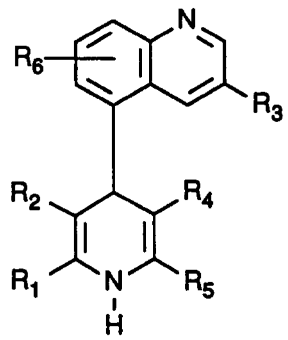

(I)

in welcher

$R^1$ und $R^5$ gleich oder verschieden sind und für geradkettiges oder verzweigtes Alkyl mit bis zu 8 Kohlenstoffatomen stehen,

$R^2$ für Nitro oder Cyano steht,

oder

$R^1$ und $R^2$ gemeinsam einen Lactonring der Formel

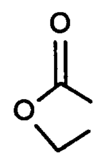

bilden,

worin

$R^6$ für Wasserstoff, Halogen oder geradkettiges oder verzweigtes Alkyl oder Alkoxy mit jeweils bis zu 8 Kohlenstoffatomen steht,

$R^3$ für Aryl mit 6 bis 10 Kohlenstoffatomen steht, das gegebenenfalls bis zu 2-fach gleich oder verschieden durch Halogen, Nitro, Cyano, Hydroxy, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder für Thienyl oder Pyridyl steht, die gegebenenfalls durch Halogen substituiert sind,

$R^4$ für Cyano, Nitro oder Formyl steht

oder

$R^4$ und $R^5$ gemeinsam einen Lactonring der Formel

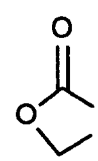

bilden,

oder

$R^4$ für eine Gruppe der Formel $-CO-A-R^8$ oder $-CO-NR^9R^{10}$ steht,

worin

A eine direkte Bindung oder ein Sauerstoffatom bedeutet,

$R^8$ Cycloalkyl mit 3 bis 8 Kohlenstoffatomen bedeutet, das gegebenenfalls durch die Gruppe $-NR^{11}$ substituiert ist, oder
einen geradkettigen, verzweigten, cyclischen, gesättigten oder ungesättigten Kohlenwasserstoffrest mit bis zu 12 Kohlenstoffatomen bedeutet, der gegebenenfalls durch Sauerstoff,

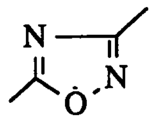

oder durch Aryliden mit 6 bis 10 Kohlenstoffatomen unterbrochen ist, wobei das Aryliden gegebenenfalls durch Halogen, Nitro, Amino, Hydroxy, Carboxy oder durch geradkettiges oder verzweigtes Alkyl, Alkoxy oder Alkoxycarbonyl mit jeweils bis zu 8 Kohlenstoffatomen substituiert ist oder der Kohlenwasserstoffrest gegebenenfalls durch Phenyl substituiert ist, das seinerseits durch Fluor, Chlor, Methyl oder Methoxy substituiert sein kann,
oder der Kohlenwasserstoffrest gegebenenfalls durch eine Gruppe der Formel $-S(O)_a$ oder $-NR^{11}$ unterbrochen ist,

a eine Zahl 0, 1 oder 2 bedeutet

und

$R^{11}$ Wasserstoff oder Aryl mit 6 bis 10 Kohlenstoffatomen bedeutet, das gegebenenfalls durch Halogen, Nitro, Phenyl oder geradkettiges oder verzweigtes Alkyl oder Alkoxy mit jeweils bis zu 4 Kohlenstoffatomen substituiert ist,
oder geradkettiges oder verzweigtes Alkyl mit bis zu 6 Kohlenstoffatomen oder Cycloalkyl mit 3 bis 8 Kohlenstoffatomen bedeutet, wobei sowohl Alkyl als auch Cycloalkyl gegebenenfalls durch Aryl mit 6 bis 10 Kohlenstoffatomen substituiert sind
und wobei der Kohlenwasserstoffrest im Falle, daß A für Sauerstoff steht, immer ein- oder zweifach gleich oder verschieden substituiert ist durch Cycloalkyl mit 3 bis 8 Kohlenstoffatomen oder durch $-CO-NR^{12}R^{13}$, $-NR^{14}-CO-R^{15}$, $-NR^{16}-SO_2-R^{17}$, $-SO_2-NR^{18}R^{19}$, $O-NO_2$, $-O-(CH_2)_b-R^{20}$, $-S(O)_c-(CH_2)_d-R^{21}$, $-NR^{22}R^{23}$ oder $-NR^{24}COOR^{25}$,

worin

$R^{12}$, $R^{13}$, $R^{14}$, $R^{15}$, $R^{16}$, $R^{17}$, $R^{18}$, $R^{19}$, $R^{24}$ und $R^{25}$ gleich oder verschieden sind und die oben angegebene Bedeutung von $R^{11}$ haben und mit dieser gleich oder verschieden sind,

b eine Zahl 1, 2, 3, 4 oder 5 bedeutet,

d eine Zahl 0, 1, 2, 3, 4 oder 5 bedeutet,

c die oben angegebene Bedeutung von a hat und mit dieser gleich oder verschieden ist,

$R^{20}$ und $R^{21}$ gleich oder verschieden sind und Aryl mit 6 bis 10 Kohlenstoffatomen bedeuten, das gegebenenfalls bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert ist,

$R^{22}$ und $R^{23}$ gleich oder verschieden sind und Wasserstoff oder einen geradkettigen, verzweigten, gesättigen, ungesättigen oder cyclischen Kohlenwasserstoffrest mit bis zu 8 Kohlenstoffatomen bedeuten, der gegebenenfalls bis zu 2-fach gleich oder verschieden durch Hydroxy, Halogen, Cycloalkyl mit 3 bis 6 Kohlenstoffatomen oder durch Aryl oder Aryloxy mit 6 bis 10 Kohlenstoffatomen substituiert ist, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können,
oder
Aryl mit 6 bis 10 Kohlenstoffatomen bedeuten, das gegebenenfalls bis zu 2-fach gleich oder

verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert ist,

oder

$R^{22}$ und $R^{23}$ gemeinsam mit dem Stickstoffatom einen 5- bis 7-gliedrigen, gesättigen oder ungesättigten Heterocyclus mit bis zu 3 Heteroatomen aus der Reihe S, N oder O bilden, der gegebenenfalls durch geradkettiges oder verzweigtes Alkyl mit bis zu 4 Kohlenstoffatomen substituiert ist, das seinerseits durch Phenyl substituiert sein kann, oder gegebenenfalls durch Phenyl substituiert ist, das seinerseits durch Halogen substituiert sein kann,

oder der Kohlenwasserstoffrest im Falle, daß A für Sauerstoff steht, substituiert ist durch einen 3- bis 7-gliedrigen gesättigten oder ungesättigten Heterocyclus oder Heterocyclyloxyring, der bis zu 3 Heteroatome aus der Reihe S, N oder O oder der Gruppe CO oder $SO_2$ enthalten kann,

wobei der Heterocyclus seinerseits bis zu 2-fach gleich oder verschieden durch Halogen, Hydroxy oder durch Aryl oder Arylsulfonyl mit 6 bis 10 Kohlenstoffatomen substituiert sein kann, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 6 Kohlenstoffatomen substituiert sein können,

oder der Heterocyclus gegebenenfalls bis zu 2-fach gleich oder verschieden durch geradkettiges oder verzweigtes Alkyl, Alkoxy oder Alkenyl mit jeweils bis zu 8 Kohlenstoffatomen substituiert ist, die ihrerseits bis zu 2-fach gleich oder verschieden durch - $NR^{26}R^{27}$ substituiert sein können,

worin

$R^{26}$ und $R^{27}$ die oben angegebene Bedeutung von $R^{22}$ und $R^{23}$ haben und mit diesen gleich oder verschieden sind,

und die gesamten Alkyl- und Alkenyl-Reste gegebenenfalls ihrerseits durch Phenyl oder Phenoxy substituiert sind, die ihrerseits bis zu 2-fach gleich oder verschieden durch Halogen, Nitro, Amino, Hydroxy, Carboxy oder durch geradkettiges oder verzweigtes Alkyl, Alkoxy oder Alkoxycarbonyl mit jeweils bis zu 4 Kohlenstoffatomen substituiert sein können,

$R^9$ und $R^{10}$ gleich oder verschieden sind und Wasserstoff, einen geradkettigen, verzweigten, cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

Aryl mit 6 bis 10 Kohlenstoffatomen oder einen 5-bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,

oder

$R^9$ und $R^{10}$ gemeinsam unter Einbezug des Stickstoffatoms einen 3-bis 8-gliedrigen, gesättigen oder ungesättigten Heterocyclus bilden, der gegebenenfalls durch Sauerstoff oder durch einen Rest der Formel $S(O)_e$, -CO- oder $-NR^{28}$ unterbrochen sein kann,

worin

e eine Zahl 0, 1 oder 2 bedeutet,

$R^{28}$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist, oder
einen cyclischen, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit 3 Heteroatomen aus der Reihe S, N oder O substituiert ist, die ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, und der gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert ist,

und deren Salze, dadurch gekennzeichnet,
daß man im Falle das $R^1$ und $R^2$ keinen Lactonring bilden,

[A] Verbindungen der allgemeinen Formel (II)

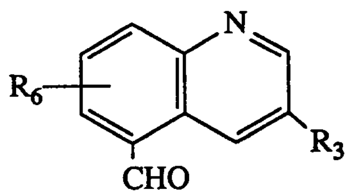

(II)

in welcher
$R^3$ und $R^6$ die oben angegebene Bedeutung haben,
zunächst mit Acylderivaten der allgemeinen Formel (III)

$R^5$-CO-$CH_2$-$R^4$ (III)

in welcher
$R^4$ und $R^5$ die oben angegebene Bedeutung haben,
gegebenenfalls unter Isolierung der entsprechenden Ylidenverbindungen der allgemeinen Formel (IV)

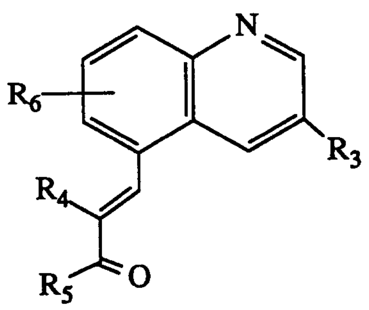

(IV)

in welcher
$R^3$, $R^4$, $R^5$ und $R^6$ die oben angegebene Bedeutung haben,
umsetzt und anschließend entweder mit Verbindungen der Formel (V)

$R^1$-CO-$CH_2$-$R^2$ (V)

in welcher
$R^1$ und $R^2$ die oben angegebene Bedeutung haben,
in Anwesenheit von Ammoniak oder Ammoniumsalzen, oder direkt mit Aminoderivaten der allgemeinen Formel (VI)

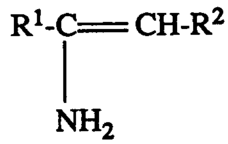

(VI)

in welcher

$R^1$ und $R^2$ die oben angegebene Bedeutung haben,

gegebenenfalls in Gegenwart von inerten organischen Lösemitteln umsetzt,

oder

[B] die Aldehyde der allgemeinen Formel (II) zunächst mit den Verbindungen der allgemeinen Formel (V), gegebenenfalls unter Isolierung der Ylidenverbindungen der allgemeinen Formel (VII)

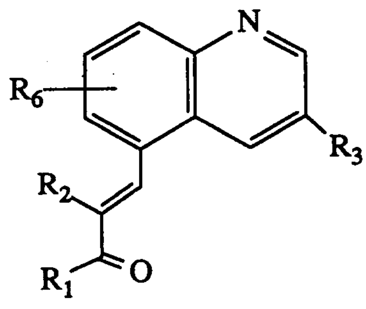

(VII)

in welcher

$R^1$, $R^2$, $R^3$ und $R^6$ die oben angegebene Bedeutung haben,

umsetzt und in einem nächsten Schritt mit den oben aufgeführten Verbindungen der allgemeinen Formel (III) in inerten Lösemitteln, in Anwesenheit von Ammoniak oder Ammoniumsalzen oder direkt mit Enaminosäurederivaten der allgemeinen Formel (VIII)

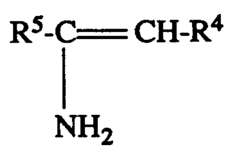

(VIII)

in welcher

$R^4$ und $R^5$ die oben angegebene Bedeutung haben,

umsetzt,

oder im Fall, daß $R^1$ und $R^2$ gemeinsam einen Lactonring bilden,

[C] zunächst nach denen unter [A] und [B] aufgeführten Methoden, Verbindungen der allgemeinen Formel (IX)

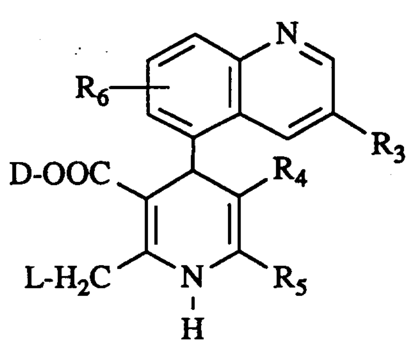

(IX)

in welcher
$R^3$, $R^4$, $R^5$ und $R^6$ die oben angegebene Bedeutung haben,
D für einen $C_1$-$C_6$-Alkyl-Rest steht
und
L für eine Abgangsgruppe wie beispielsweise Chlor oder Acetoxy steht,
herstellt und nach bekannten Verfahren einen säure- oder basenkatslysierten Ringschluß anschließt,
oder
[D] im Fall, daß $R^4$ für den Rest der Formel -CO-A-$R^8$ (A = Sauerstoff) oder -CO-$NR^9R^{10}$ steht, Verbindungen der allgemeinen Formel (X)

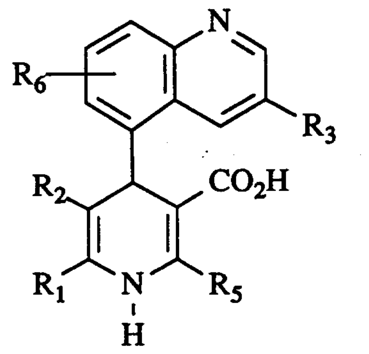

(X)

in welcher
$R^1$, $R^2$, $R^3$ $R^5$, und $R^6$ die oben angegebene Bedeutung haben,
gegebenenfalls über ein reaktives Säurederivat mit Alkoholen oder Aminen der allgemeinen Formel (XI) oder (XIII)

HA - $R^8$ (XI)

oder

$HNR^9R^{10}$ (XII)

in welcher
A, $R^8$, $R^9$ und $R^{10}$ die oben angegebene Bedeutung haben,
umsetzt, wobei bei Einsatz chiraler Carbonsäuren die entsprechenden chiralen Ester oder Amide erhalten werden.

6. Verbindungen der allgemeinen Formel (I) gemäß Anspruch 1 zur Verwendung bei der Behandlung von Herz-Kreislauferkrankungen.

7. Arzneimittel enthaltend mindestens eine Verbindung der allgemeinen Formel (I) gemäß Anspruch 1.

8. Verfahren zur Herstellung von Arzneimitteln, dadurch gekennzeichnet, daß man mindestens eine Verbindung der allgemeinen Formel (I) gemäß Anspruch 1 gegebenenfalls mit üblichen Hilfs- und Trägerstoffen in eine geeignete Applikationsform überführt.

9. Verwendung von Verbindungen der allgemeinen Formel (I) gemäß Anspruch 1 in Arzneimitteln mit positiv inotroper Wirkung.

Europäisches Patentamt

# EUROPÄISCHER RECHERCHENBERICHT

Nummer der Anmeldung
EP 94 10 5775

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,X | EP-A-0 452 712 (BAYER AG)<br>* das ganze Dokument *<br>--- | 1,6 | C07D491/04<br>A61K31/47<br>C07D401/04<br>C07D401/14<br>C07D417/14<br>//(C07D491/04,<br>307:00,221:00) |
| A | EP-A-0 518 105 (BAYER AG)<br>* Ansprüche *<br>--- | 1,6 | |
| A | EP-A-0 515 940 (BAYER AG)<br>* Ansprüche *<br>----- | 1,6 | |
| | | | RECHERCHIERTE SACHGEBIETE (Int.Cl.5)<br>C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 1. August 1994 | Van Bijlen, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument